Europäisches Patentamt

European Patent Office

Office européen des brevets

(11) Numéro de publication : **0 507 677 A1**

(12) **DEMANDE DE BREVET EUROPEEN**

(21) Numéro de dépôt : 92400911.1

(22) Date de dépôt : 01.04.92

(51) Int. Cl.⁵ : **C07D 251/54**

(30) Priorité : **04.04.91 FR 9104086**

(43) Date de publication de la demande :
**07.10.92 Bulletin 92/41**

(84) Etats contractants désignés :
**AT BE CH DE DK ES FR GB GR IT LI LU NL PT SE**

(71) Demandeur : **ELF ATOCHEM S.A.**
**4 & 8, Cours Michelet La Défense 10**
**F-92800 Puteaux (FR)**

(72) Inventeur : **Paul, Jean-Michel**
**99 Ancienne Route Impériale**
**F-31120 Portet/Garonne (FR)**

(74) Mandataire : **Rieux, Michel et al**
**ELF ATOCHEM S.A., Département Propriété**
**Industrielle, La Défense 10, Cédex 42**
**F-92091 Paris La Défense (FR)**

(54) **Procédé de synthèse du cyanurate de mélamine.**

(57) Procédé de synthèse de cyanurate de mélamine par réaction d'acide cyanurique et de mélamine en milieu aqueux, effectué en présence d'un acide minéral fort à pH au plus égal à 1.

EP 0 507 677 A1

Jouve, 18, rue Saint-Denis, 75001 PARIS

La présente invention concerne un procédé de fabrication de cyanurate de mélamine , en milieu aqueux, à partir d'acide cyanurique et de mélamine et en présence d'un acide minéral fort.

Plusieurs voies de synthèse ont, par le passé, été proposées pour fabriquer le cyanurate de mélamine.

Ainsi, il est connu de fabriquer le cyanurate de mélamine par réaction d'acide cyanurique et de mélamine en milieu aqueux. Selon le brevet JP 56032470, on procédé à pH supérieur à 7, au lieu de pH 3 à 12 selon le brevet PL 100877.

La synthèse en milieu aqueux peut être menée à froid (brevet JP 54055588-A) : elle est alors très lente. Un art antérieur plus important concerne des procédés à chaud. C'est le cas du brevet JP 55147266-A qui préconise, en fin de réaction, une élimination de l'eau par concentration sous vide. De même, le brevet JP 54141792-A a pour objet un procédé de synthèse à 90° C selon lequel on récupére le produit final par filtration puis séchage à 105° C.

D'autre part, le brevet JP 5405587 divulgue un procédé de fabrication de cyanurate de mélamine par réaction d'acide cyanurique et de mélamine en présence d'eau dans un malaxeur, pendant 4 heures à 90° C.

Enfin, le brevet JP 54125690-A concerne un procédé de synthèse de cyanurate de mélamine par mélange d'acide cyanurique et de mélamine à l'état solide, puis chauffage à 200° C. Cette dernière technique présente l'inconvénient de nécessiter des réacteurs munis de moyens de malaxage puissants.

Les synthèses en milieu aqueux connues jusqu'à présent sont conduites à pH supérieur à 3, et nécessitent de grandes quantités d'eau. En effet, une dilution insuffisante aboutit à l'obtention de pâtes, imposant l'utilisation de malaxeurs. L'élimination de telles quantités d'eau, en fin de réaction, peut devenir une limite rédhibitoire de ces procédés, entre autres en raison de sa durée excessive.

De plus, le cyanurate de mélamine obtenu par ces procédés en milieu aqueux a une granulométrie très fine, de sorte que sa filtration par les moyens simples conduit à un colmatage des filtres, à moins d'utiliser des techniques de filtration plus compliquées, comme la filtration tangentielle. Ce produit final est en outre très difficile à laver et contient des impuretés, essentiellement constituées d'acide cyanurique et de mélamine n'ayant pas réagi.

La demanderesse a maintenant trouvé un procédé ne présentant aucun de ces inconvénients.

La présente demande a pour objet un procédé de synthèse de cyanurate de mélamine par réaction d'acide cyanurique et de mélamine en milieu aqueux, caractérisé en ce qu'il est mis en oeuvre à pH au plus égal à 1, en présence d'un acide minéral fort.

Le rapport molaire acide cyanurique/mélamine est compris entre 0,7 et 1,4, et de préférence égal à 1.

Pour 100 parties en poids d'acide cyanurique et de mélamine, on met en oeuvre au moins 70 parties en poids d'eau, et de préférence au moins 120 parties en poids d'eau.

Le rapport molaire $[H^+]$ / [mélamine] est au moins égal à 0,5, et de préférence compris entre 1 et 1,2.

La réaction peut être mise en oeuvre à une température comprise entre 20 et 98° C sous pression atmosphérique.

On peut également effectuer la synthèse sous pression.

De préférence, on opère entre 80 et 95° C sous pression atmosphérique pendant 10 à 30 minutes.

Après la réaction, le milieu réactionnel est filtré à une température comprise entre 20 et 80° C, et de préférence entre 50 et 70° C.

Le gâteau de filtration est ensuite lavé à grande eau à une température comprise entre 20 et 80° C, de préférence entre 50 et 70° C.

Enfin, le gâteau ainsi recueilli est séché en étuve à 120°C ou dans un four à micro-ondes.

Tout acide minéral fort peut être employé. Cependant, l'acide chlorhydrique est préféré, car son utilisationconduit à la formation de sels de mélamine solubles, en particulier de chlorhydrates $-NH_3^+Cl^-$.

Le procédé objet de la présente invention supprime en effet tous les inconvénients connus des procédés en milieu aqueux.

Le milieu n'est pas épais, même à forte concentration d'acide cyanurique et de mélamine; l'agitation est très facile, et la réaction peut être effectuée dans un simple réacteur agite.

L'emploi de faibles quantités d'eau est permis. La vidange du réacteur est de ce fait plus rapide.

La filtration du cyanurate de mélamine est aisée, même par les moyens les plus simples. Le produit final est en effet constitué de grains plus gros que ceux obtenus par les procédés connus. Leur répartition granulométrique est conforme à une courbe de Gauss centrée sur 12 microns.

Le produit recueilli par filtration peut être lavé très facilement, et débarassé de ses impuretés, telles l'acide cyanurique ou la mélamine résiduels. Ce procédé s'avère d'une grande souplesse; il permet de travailler avec un excès d'acide cyanurique ou de mélamine, qui seront éliminés au lavage.

La pureté du cyanurate de mélamine obtenu est supérieure à 99,7 % en poids.

Les exemples suivants illustrent la présente invention. Tous les pourcentages y sont exprimés en poids.

Exemple 1 :

Dans un réacteur agité mécaniquement (agitation type ancre) et chauffe par double enveloppe on introduit successivement de l'eau puis 0,5 mole d'acide chlorhydrique. On porte alors la température du milieu réactionnel à 75° C puis on introduit 0,5 mole d'acide cyanurique et 0,5 de mole mélamine sous agitation.

Pour chaque essai on a mis en oeuvre les quantités d'eau suivantes :

| N° Essai | Quantité Eau (g) |
|----------|------------------|
| 1 | 500 |
| 2 | 392 |
| 3 | 209 |
| 4 | 187,5 |

On laisse 15 minutes sous agitation à une température comprise entre 80 et 90° C.

Le mélange réactionnel est ensuite refroidi à 50° C, filtré sur fritté n° 3 puis lavé avec de l'eau à 50° C.

Le produit final est séché en étuve à 120° C.

| N° essai | $\dfrac{AC+MEL}{Eau} \times 100$ (%) | Aspect du Milieu en fin de réaction | Analyse du MCA | | | | Rendements MCA produit MCA théorique (%) |
|---|---|---|---|---|---|---|---|
| | | | (AC+Mel) par ATG% | HPLC % | | Cl⁻ PPm | |
| | | | | AC | Mel | | |
| 1 | 25,5 | très fluide | < 0,5 | 0,10 | 0,11 | <10 | 97,7 |
| 2 | 32,5 | très fluide | < 0,5 | 0,15 | 0,10 | <10 | 98,0 |
| 3 | 61 | fluide | < 0,5 | 0,15 | 0,08 | <10 | 96,7 |
| 4 | 68 | fluide | < 0,5 | 0,15 | 0,15 | <10 | 97,5 |

AC : Acide Cyanurique; Mel : Mélamine;
MCA : Cyanurate de Mélamine
ATG : Analyse Thermogravimétrique

Exemple 2 : **Les exemples 2 à 7 sont réalisés en mettant en oeuvre 500 g d'eau.**

L'exemple 1 est repris en neutralisant le milieu réactionnel à la soude avant filtration/lavage.

On obtient un produit sec dont la pureté quoique bonne, renferme un peu d'Ammeline.

|  | Pas de Neutralisation avant filtration | Neutralisation avant filtration |
|---|---|---|
|  | Analyse HPLC (%) | Analyse HPLC (%) |
|  | essai n° 4 | essai n° 5 |
| AC | 0,10 | 0,12 |
| Mel | 0,10 | 0,15 |
| Ade | < 0,01 | < 0,01 |
| Ane | < 0,01 | 0,35 |

Ade : Ammelide; Ane : Ammeline

Exemple 3 :

On fait varier le rapport molaire Hcl/Mélamine. Les autres conditions sont celles de l'exemple n°1.

| N° es-sai | [Hcl]/[Mel] | Aspect du produit final | Rende ment (%) | Analyses | |
|---|---|---|---|---|---|
|  |  |  |  | ATG (%) | HPLC (%) |
| 6 | 0,1 | dur | 88,4 | AC=12 Mel=1 | |
| 7 | 0,5 | dur | 85 | AC=17 Mel=3 | |
| 8 | 1 | très friable | 97 | AC+Mel <0,5 | AC=0,10 Mel=0,10 |
| 9 | 1,1 | très friable | 97,2 | AC+Mel <0,5 | AC=0,11 Mel=0,12 |
| 10 | 1,5 | très friable | 97 | AC+Mel <0,5 | AC=0,12 Mel=0,15 |

Exemple 4 :

On reproduit un essai sous les conditions décrites à l'exemple 1. Pour les essais suivants, numérotés n, on utilise les eaux de filtration riches en HCl provenant de l'essai n° (n-1), et l'on complète avec de l'HCl pur afin d'ajuster le rapport molaire [HCl]/[Mel] à une valeur de 1.

EP 0 507 677 A1

| N° essai | Quantité d'HCl récupéré dans le filtrat | | Rende ment(%) MCA | Analyses du produit sec | | |
|---|---|---|---|---|---|---|
| | Moles | Taux de Récupération de HCl (%) | | ATG (%) (AC+MEL | HPLC (%) | Cl⁻ PPm) |
| 11 | 0,47 | 78 | 96 | < 0,5 | AC=0,16Mel=0,12 | < 10 |
| 12 | 0,40 | 67 | 97 | < 0,5 | AC=0,18Mel=0,16 | < 10 |
| 13 | 0,40 | 67 | 97 | < 0,5 | AC=0,18Mel=0,20 | |
| 14 | 0,44 | 67,6 | 97 | < 0,5 | AC=0,19Mel=0,13 | |
| 15 | 0,46 | 75 | 97 | < 0,5 | AC=0,10Mel=0,10 | |

Exemple 5 :

On reproduit l'exemple 1 en faisant varier les températures de filtration.

| Température de filtration | 50° C | | 60° C | | 70° C | |
|---|---|---|---|---|---|---|
| n° essai | 16 | | 17 | | 18 | |
| | rende ment MCA % | HPLC % | rende ment MCA % | HPLC % | rende ment MCA % | HPLC % |
| | 96,7 | AC=0,16 Mel=0,12 | 96,6 | AC=0,20 Mel=0,14 | 96,4 | AC <0,02 Mel <0,04 |

Exemple 6 :

On réalise la synthèse du cyanurate de mélamine en reprenant les conditions de l'exemple 1, à ceci près que l'on introduit dans le réacteur un excès de mélamine, puis un excès d'acide cyanurique.

5

| n° essai | rapport molaire [Mel]/[AC] | Composition des eaux de filtration (grammes) | | | | rende ment MCA % | Analyses du MCA % | |
|---|---|---|---|---|---|---|---|---|
| | | Mel | AC | Ade | Ane | | HPLC (%) | Cl⁻ ppm |
| 19 | 1,1 | 4 | 2,7 | 0,9 | 0 | 98,8 | AC=0,13 Mel=0,13 | 20 |
| 20 | 0,90 | 1,1 | 5,0 | 0,12 | 0,37 | 97 | AC=0,10 Mel=0,14 | 10 |

Cet exemple montre que l'excès d'un des deux réactifs de départ est évacué dans les eaux de filtration.

D'autre part, la répartition granulométrique laser du cyanurate de mélamine se présente sous forme d'une courbe de Gauss centrée sur 12 microns.

Exemple 7 :

L'exemple 1 est repris en remplaçant Hcl à $H_2SO_4$. Le milieu réactionnel est plus épais.

L'analyse HPLC ,du cyanurate de mélamine indique les pourcentages suivants :

AC = 0,07 %

Mel = 0,16 %

Ane = 90 ppm

$SO_4^{2-}$= 900 ppm

Les ions sulfate semblent s'extraire plus difficilement du cyanurate de mélamine par lavage que les ions chlorure.

Exemple 8 :

On opère en réacteur émaillé agité mécaniquement de 1 m³. Le chauffage du réacteur est effectué avec de la vapeur sous une pression de 3 bars. On charge 800 l d'eau puis 89,7 Kg d'HCl à 36,7 % en poids; on chauffe à 75° C puis on introduit 100,8 Kg de mélamine et 103,2 Kg d'acide cyanurique . On maintient à 90 - 93° pendant 10 minutes; On refroidit ensuite à 50° C et on filtre sur buchner (toile de filtration en polypropylène de porosité = 2 microns). Le gâteau est lavé avec 700 l d'eau à 50° C et séché en étuve à 120° C.

| n° essai | Aspect du MCA sec | rendement MCA % | Analyses du MCA | | |
|---|---|---|---|---|---|
| | | | HPLC % | Cl⁻ ppm | granulométrie |
| 22 | blanc | 96,5 | AC= 0,13 Mel=0,14 | <10 | centrée sur 12microns |

Exemple 9 :

On opère dans le même réacteur qu'à l'exemple précédent, en diminuant le rapport massique eau/(AC+Mel). En effet, on introduit dans le réacteur 350 Kg d'eau, 64,5 Kg d'acide cyanurique, 63 Kg de mélamine et 56 Kg d'HCl à 36,1 % en poids.

La réaction est effectuée à 90 - 93° C pendant 10 minutes, le filtration à 50° C, le lavage avec 700 litres d'eau à 50° C.

| n° essai | Rendement % MCA | Analyse du MCA | | |
|---|---|---|---|---|
| | | HPLC (%) | Cl- ppm | Granulométrie |
| 23 | 95 | AC=0,11 Mel=0,12 | <10 | Centrée sur 12 microns |

Dans cet exemple, on a augmenté la productivité en diminuant le volume de réaction et la quantité d'eau. On observe une diminution faible du rendement.

## Revendications

1. Procédé de synthèse de cyanurate de mélamine par réaction d'acide cyanurique et de mélamine en milieu aqueux, caractérisé en ce qu'il est mis en oeuvre à pH au plus égal à 1, en présence d'un acide minéral fort.

2. Procédé selon 1, caractérisé en ce que le rapport molaire acide cyanurique/mélamine est compris entre 0,7 et 1,4.

3. Procédé selon 2, caractérisé en ce que le rapport molaire acide cyanurique/mélamine est égal à 1.

4. Procédé selon 2, caractérisé en ce que pour 100 parties en poids d'acide cyanurique et de mélamine, on met en oeuvre au moins 70 parties en poids d'eau.

5. Procédé selon 4, caractérisé en ce que pour 100 parties en poids d'acide cyanurique et de mélamine, on met en oeuvre au moins 120 parties en poids d'eau.

6. Procédé selon 4, caractérisé en ce que le rapport molaire $[H^+]/[melamine]$ est au moins égal à 0,5.

7. Procédé selon 6, caractérisé en ce que le rapport molaire $[H^+]/[mélamine]$ est compris entre 1 et 1,2.

8. Procéde selon 6, caractérise en ce que la réaction est mise en oeuvre sous pression atmosphérique à une température comprise entre 20 et 98° C.

9. Procédé selon 8, caractérisé en ce que la réaction est mise en oeuvre à une température entre 80 et 95° C pendant 10 à 30 minutes.

10. Procédé selon 6, caractérisé en ce que la synthèse est effectuée à une pression supérieure à la pression atmospheriqué.

11. Procédé selon 8 ou 10, caractérisé en ce que, après la réaction, le milieu réactionnel est filtré à une température comprise entre 20 et 80°C.

12. Procédé selon 11, caractérisé en ce que, après la réaction, le milieu réactionnel est filtré à une température comprise entre 50 à 70°C.

13. Procédé selon 11, caractérisé en ce que le gâteau de filtration est lavé à l'eau à une température comprise entre 20 et 80°C.

14. Procédé selon 13, caractérisé en ce que la température de l'eau de lavage est comprise entre 50 et 70°C.

15. Procédé selon 13, caractérisé en ce que le gâteau de filtration est finalement séché en étuve à 120°C ou dans un four à micro-ondes.

16. Procédé selon 15, caractérisé en ce qe l'acide minéral fort est l'acide chlorhydrique.

**European Patent Office**

# EUROPEAN SEARCH REPORT

Application Number

EP    92 40 0911

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | GB-A-2 030 594 (MITSUBUSHI PETROCHEMICAL CO. LTD.) <br> * page 2, line 7-13; page 6, line 3-13 * <br> --- | 1,3,5,9 | C07D251/54 |
| D,A | CHEMICAL ABSTRACTS, vol. 92, <br> 1980, Columbus, Ohio, US; <br> abstract no. 111070S, <br> WOJTOWICZ,TADEUSZ ET AL.: 'Melamine cyanurate' <br> page 670 ; <br> & Pol. 100,877 <br> * abstract * <br> --- | 1,5,6 | |
| A | CHEMICAL ABSTRACTS, vol. 91, <br> 1979, Columbus, Ohio, US; <br> abstract no. 193339C, <br> MATSUKI, KAZUO ET AL.: 'Melamine cyanurate' <br> page 652 ; <br> & Jpn. Kokai Tokkyo Koho 79 55,587 <br> * abstract * <br> --- | 1,5,6 | |
| A | CHEMICAL ABSTRACTS, vol. 97, <br> 1982, Columbus, Ohio, US; <br> abstract no. 91335E, <br> RUKEVICH,O.S. ET AL.: 'Kinetic study of the synthesis of melamine cyanurate' <br> page 689 ; <br> & Deposited Doc. 1980, SPSTL 974 khp-D80 <br> * abstract * <br> ----- | 1,9 | **TECHNICAL FIELDS SEARCHED (Int. Cl.5)** <br><br> C07D |

The present search report has been drawn up for all claims

| Place of search | Date of completion of the search | Examiner |
|---|---|---|
| THE HAGUE | 07 JULY 1992 | VAN BIJLEN H. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................................................
& : member of the same patent family, corresponding document

EPO FORM 1503 03.82 (P0401)